# EUROPEAN PATENT SPECIFICATION

(11) **EP 4 524 127 B1**
(45) Date of publication and mention of the grant of the patent: **11.11.2026**
(21) Application number: 22941032.9
(22) Date of filing: 09.05.2022
(51) Int. Cl.: C07C 201/12, C07C 205/45, A01N 37/34, A01P 13/00, C07C 41/01, C07C 253/30, C07C 255/41

(54) **METHOD FOR PREPARING 4-NITRO-2-TRIFLUOROMETHYL ACETOPHENONE AND USE THEREOF**
VERFAHREN ZUR HERSTELLUNG VON 4-NITRO-2-TRIFLUORMETHYLACETOPHENON UND DESSEN VERWENDUNG
PROCÉDÉ DE PRÉPARATION DE 4-NITRO-2-TRIFLUOROMÉTHYL ACÉTOPHÉNONE ET SON UTILISATION

(43) Date of publication of application: 19.03.2025
(73) Proprietor: Max(Rudong) Chemicals Co Ltd, Nantong, Jiangsu 361012 (CN)
(72) Inventor: CHEN, Bangchi, Hangzhou, Zhejiang 310052 (CN); SUN, Yinwei, Hangzhou, Zhejiang 310052 (CN); WANG, Zhongyuan, Hangzhou, Zhejiang 310052 (CN); SU, Yehua, Hangzhou, Zhejiang 310052 (CN); XU, Xiaoyan, Hangzhou, Zhejiang 310052 (CN); MU, Haiping, Hangzhou, Zhejiang 310052 (CN)
(74) Representative: Haseltine Lake Kempner LLP
(86) International application number: PCT/CN2022/091715
(87) International publication number: WO 2023/216064

(56) References cited:
- EP-A1- 3 670 492
- WO-A1-2021/160468
- WO-A1-2021/160468
- CN-A- 105 777 580
- CN-A- 112 409 142
- CHEN YING, XU LANTING, JIANG YONGWEN, MA DAWEI: "Assembly of α‐(Hetero)aryl Nitriles via Copper‐Catalyzed Coupling Reactions with (Hetero)aryl Chlorides and Bromides", ANGEWANDTE CHEMIE INTERNATIONAL EDITION, VERLAG CHEMIE, HOBOKEN, USA, vol. 60, no. 13, 22 March 2021 (2021-03-22), Hoboken, USA, pages 7082 - 7086, XP093106823, ISSN: 1433-7851, DOI: 10.1002/anie.202014638

## Description

### TECHNICAL FIELD

This application relates to organic synthesis, and more specifically to a method for preparing 4-nitro-2-(trifluoromethyl)acetophenone and an use thereof.

### BACKGROUND

4-Nitro-2-(trifluoromethyl)acetophenone is an important organic compound. It is a key intermediate for preparing fungicide mefentrifluconazole (EP3670491A1). Therefore, it is of great significance to prepare such compound simply and efficiently.

European patent Publication No. 3670491A1 discloses a preparation of 4-nitro-2-(trifluoromethyl)acetophenone, where 4-nitro-2-(trifluoromethyl)chloroben zene and nitroethane are successively subjected to nucleophilic substitution and oxidation to produce the 4-nitro-2-(trifluoromethyl)acetophenone, as shown in Scheme 1. The application of this method is greatly limited because of the dangerous raw material nitroethane.

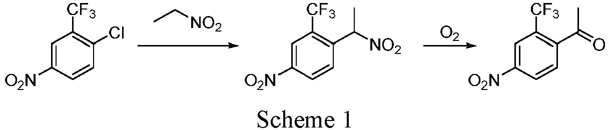

International Patent Publication Nos. WO2021160468 and WO2021047978 disclose another preparation route of the 4-nitro-2-(trifluoromethyl)acetophenone, in which α-methyl-2-(trifluoromethyl)-benzyl alcohol undergoes nitration under the action of a mixed acid, followed by reaction in the presence of a base to give the 4-nitro-2-(trifluoromethyl)acetophenone, as shown in Scheme 2. This method required the use of mixed acid, resulting in the generation a large amount of waste acid. Meanwhile, this method required the use of an environmentally-unfriendly halogenated alkane solvent dichloroethane (DCE). The intermediate nitrate is an impact-sensitive substance (WO 2021047978), possessing significant safety risks.

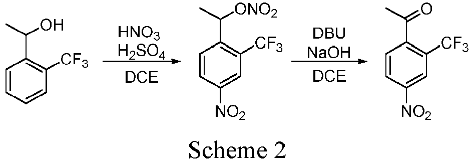

The inventors of this application discovered a new method for preparing 4-nitro-2-(trifluoromethyl)acetophenone through design and exploration. This method is safer, higher yielding, and more amenable for industrial production.

### SUMMARY

A first object of this application is to provide a method for preparing 4-nitro-2-(trifluoromethyl)acetophenone of formula (I), including:
(i) reacting a compound (II) with a compound (III) in the presence of a base A to obtain a compound (IV);
(ii) converting the compound (IV) into a compound (V) in the presence of a base B; and
(iii) in the presence or absence of a base C, reacting the compound (V) with an oxidant to obtain the compound of formula (I); as shown in the following reaction scheme: 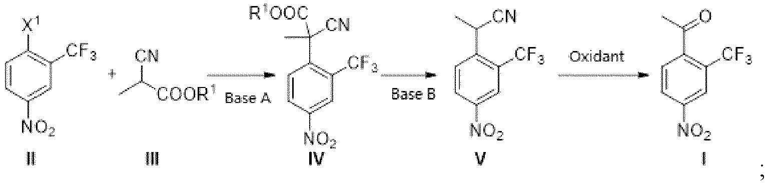 wherein X¹ is halogen; R¹ is hydrogen, a C₁-C₁₀ alkyl group, a C₆-C₁₂ aryl group, or a heteroaryl group containing one or two atoms selected from nitrogen, oxygen, and sulfur; and the alkyl group, aryl group and heteroaryl group are unsubstituted or substituted with halogen; preferably, R¹ is hydrogen, a C₁-C₆ alkyl group, or a C₁-C₆ halogenated alkyl group, and more preferably, R¹ is a C₁-C₄ alkyl group or a C₁-C₄ halogenated alkyl group.

The base A, the base B, or the base C are each independently selected from the group consisting of an alkali metal carbonate, an alkali metal bicarbonate, an alkali metal phosphate, an alkali metal C₁-C₆ alkyl carboxylate, an alkali metal formate, an alkali metal hydroxide, an alkali metal alcoholate, an alkali metal hydride, an alkaline earth metal carbonate, an alkaline earth metal bicarbonate, an alkaline earth metal phosphate, an alkaline earth metal C₁-C₆ alkyl carboxylate, an alkaline earth metal formate, an alkaline earth metal hydroxide, an alkaline earth metal alcoholate, an alkaline earth metal hydride, an alkali metal alkylide, an alkali metal amide compound, an organic amine compound, and a mixture thereof; preferably, the base A, the base B, and the base C are each independently selected from the group consisting of an alkali metal carbonate, an alkali metal phosphate, an alkali metal hydroxide, an organic tertiary amide compound (such as substituted or non-substituted pyridine compounds), and a mixture thereof; and more preferably, the base A, the base B, and the base C are each independently selected from the group consisting of potassium carbonate, potassium hydroxide, sodium carbonate, sodium hydroxide, an alkyl tertiary amine compound (such as cyclic alkyl tertiary amine), and a mixture thereof; where the alkyl group is C₁-C₁₂ alkyl group, preferably C₁-C₆ alkyl group; and

The oxidant is selected from the group consisting of oxygen, air, ozone, peroxide compound, hypohalite, halate, perhalogenate, and a mixture thereof.

The peroxide compound is selected from the group consisting of metal peroxide, hydrogen peroxide, peroxyacid salt, and organic peroxide, such as hydrogen peroxide, sodium peroxide, potassium peroxide, calcium peroxide, magnesium peroxide, zinc peroxide, potassium peroxymonosulfate, and strontium peroxide.

The hypohalite is selected from the group consisting of calcium hypochlorite, lithium hypochlorite, sodium hypochlorite, and potassium hypochlorite.

The halate is selected from the group consisting of sodium chlorate, sodium bromate, and potassium iodate.

The perhalogenate is selected from the group consisting of ammonium perchlorate salt, potassium perchlorate, sodium perchlorate and perchloric acid.

In an embodiment, the oxidant is oxygen or air.

In an embodiment, a molar ratio of the compound (III) to the compound (II) is (1.0~3.5): 1, preferably (1.0~2.5): 1, and more preferably (1.0~2.0): 1.

In an embodiment, a molar ratio of the compound (III) to the base A is (0.5~2.0): 1, preferably (0.5~1.5): 1, and more preferably (0.9~1.1): 1.

In an embodiment, a molar ratio of the compound (IV) to the base B is (0.1~2): 1, preferably (0.5~1.5): 1, and more preferably (0.5~1.0): 1.

In an embodiment, a molar ratio of the compound (V) to the base C is 1: (0.01~3), preferably 1: (0.01~2), and more preferably 1: (0.1~2.0).

In an embodiment, the steps (i)-(iii) may be independently carried out in the presence of solvents. The solvent is selected from the group consisting of nitriles, esters, haloalkanes, ethers, aromatic hydrocarbons, tertiary amines, amides, sulfones and sulfoxides, water, alcohols, ketones, and a mixture thereof; preferably amides, sulfones and sulfoxide compounds, and a mixture thereof; and further preferably dimethylformamide, N-methylpyrrolidone, dimethylacetamide, dimethyl sulfoxide, and a mixture thereof.

In an embodiment, the steps (i)-(iii) may be independently carried out under solvent-free conditions.

In an embodiment, the steps (i)-(iii) may react independently in the temperature range of -20 to 150°C, preferably 20 to 100°C, more preferably 30 to 80°C, and further preferably 50 to 80°C.

In an embodiment, the step (i) is carried out in the presence of a catalyst A. The catalyst A is selected from the group consisting of transition elements in groups VIII, IB and IIB, and cation thereof, and a mixture thereof.

In an embodiment, the step (iii) is carried out in the presence of a catalyst B. The catalyst B is selected from the group consisting of transition elements in groups VIII, IB and IIB, and cation thereof, and a mixture thereof.

In an embodiment, the VIII-group transition element includes iron, cobalt, nickel, ruthenium, rhodium, palladium, osmium, iridium, and platinum; the IB-group transition elements include copper, silver, and gold; and the IIB-group transition elements include zinc and cadmium.

In an embodiment, the catalyst A is an IB-group transition element or cation thereof, preferably elemental copper or Cu⁺ or Cu²⁺, and a mixture thereof.

In an embodiment, a molar ratio of the catalyst A to the compound (III) is (0.01~ 1.0): 1, preferably (0.01~0.1): 1, and more preferably (0.01~0.05): 1.

In an embodiment, the catalyst B is a VIII-group transition element or an IB-group transition element or cation thereof, preferably elemental copper, elemental iron, metallic copper cation, metallic iron cation or a mixture thereof, and further preferably elemental copper or Cu⁺ or Cu²⁺, and a mixture thereof. The metal cation salt includes halide, haloate, sulfate, bisulfate, nitrate, C₁-C₁₀ carboxylate, carbonate, phosphate, monohydrogen phosphate, dihydrogen phosphate, pyrophosphate, carbonyl sulfonate, fluoroborate, hydroxide, and oxide.

In an embodiment, a molar ratio of the catalyst B to the compound (V) is (0.01 ~ 1.0): 1, preferably (0.01 ~ 0.2): 1, and more preferably (0.05 ~ 0.15): 1.

In an embodiment, the method of preparing the compound (I) is carried out in a "one-pot" manner. The compound (II), compound (III), and the base A are mixed to react, and without treatment, the mixture is directly reacted with an oxidant to obtain compound (I).

In an embodiment, the method of preparing the compound (I) is carried out in a "one-step" manner. The compound (II), the compound (III), the base A and oxidant are directly mixed to prepare the compound (I), as shown in the following reaction scheme: 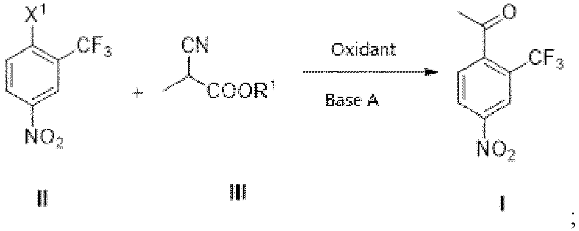 where X¹, R¹, the base A and the oxidant are defined as above.

This application provides use of the compound (IV) as a herbicide, which can effectively inhibit the growth of weeds of the gramineae and the broadleaved, such as in the control of *Solanum nigrum L., Chenopodium album L., Echinochloa crusgalli (L.) Beauv, and Panicum acroanthum Steud.*

This application further provides use of the compound (I). The compound (I) is reacted with p-chlorophenol react in the presence of a base D to prepare 1-(4-(4-chlorophenoxy)-2-trifluoromethylphenyl)ethan-1-one, as shown in following reaction scheme:

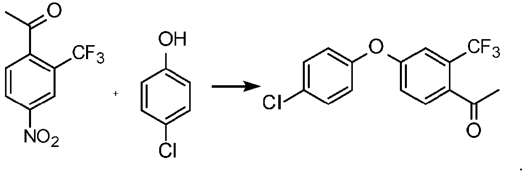

In an embodiment, the base D is the alkali metal carbonate or the alkaline earth metal carbonate, and preferably sodium carbonate and potassium carbonate.

As used herein, the terms "comprising, including, or having" is non-exclusive. For example, a process or method includes some elements, but not limited to those elements, may also include other elements that are not listed.

As used herein, unless otherwise expressly specified and defined, "or" is non-exclusive. For example, "A or B" indicates that A is true (or exists) and B is false (or does not exist), A is false (or does not exist) and B is true (or exists), and both A and B are true (or exist). The "mixing" means an operation to allow mutual contact of the chemicals.

In this application, the alkyl group may be linear or branched. The term "halogen", either alone or in the compound words such as "halogenated alkyl", includes fluorine, chlorine, bromine or iodine. Further, when used in compound words such as "halogenated alkyl", the alkyl group may be partially or wholly replaced by halogen atoms that may be the same or different. Halogenated alkyl may be F₃C, ClCH₂, CF₃CH₂, or CF₃CC₁₂.

Compared to the known method for preparing 4-nitro-2-(trifluoromethyl)acetophenone, the method provided in this application uses readily available raw materials, avoids the use of dangerous raw materials and intermediates. The method is safe to operate, easier to be carried out in industrial production.

Further, when the "one-pot" or "one-step" reaction is used, the steps of the method of the application are shorter and the process simpler.

Further, when a transition metal catalyst is added in the step (iii), the reaction yields of the method provided by the application are higher. The reaction can be carried out in absence of base. The wastes are less and overall costs are lower.

It is worthwhile to point it out that the compound (IV) provided herein has biological herbicidal activity against weeds such as *Solanum nigrum L., Chenopodium album L., Echinochloa crusgalli (L.) Beauv, and Panicum acroanthum Steud.* Specifically, it can inhibit the emergence and growth of weeds.

### DETAILED DESCRIPTION OF EMBODIMENTS

This application will be described in detail below with reference to the embodiments to make objects, technical features and advantages of this application clearer, but these embodiments are not intended to limit the scope of this application.

### Example 1 Preparation of 1-(4-nitro-2-trifluoromethylphenyl)ethan-1-one

### Step (1) Preparation of ethyl 2-(4-nitro-2-trifluoromethylphenyl)-2-cyanopropanoate

To a 100 mL reaction flask were sequentially added 3.4 g of ethyl 2-cyanopropionate, 3.1 g of potassium carbonate, 5 g of 2-trifluoromethyl-4-nitro-chlorobenzene, 0.1 g of cuprous chloride, and 20 mL of N, N-dimethylformamide. The reaction mixture was heated to 50°C in an oil bath, reacted at 50°C for 8 h, cooled, and added to 150 mL of toluene. Then, the obtained reaction solution was washed and concentrated to obtain 6.7 g of ethyl 2-(4-nitro-2-trifluoromethylphenyl)- 2-cyanopropanoate (95% yield).

¹H NMR (CDCl₃, 500 MHz, TMS): δ 8.62 (d, *J =* 2.0 Hz, 1H), 8.50 (dd, *J₁* = 9.0 Hz, *J₂* = 2.0 Hz, 1H), 8.03 (d, *J =* 9.0 Hz, 1H), 4.32 (q, *J =* 7.0 Hz, 2H), 2.22 (s, 3H), 1.31 (t, *J =* 7.0 Hz, 3H).

¹³C NMR (CDCl₃, 125 MHz): δ 166.9, 147.7, 139.0, 130.5, 129.9 (q, *J =* 26 Hz), 126.8, 124.0 (q, *J =* 4.4 Hz), 122.7 (q, *J =* 218.5 Hz), 117.6, 63.9, 46.9, 25.1, 13.4.

### Step (2) Preparation of 2-(4-nitro-2-trifluoromethylphenyl)propionitrile

To a 100 mL reaction flask were sequentially added 6.7 g of ethyl 2-(4-nitro-2-trifluoromethylphenyl)-2-cyanopropanoate prepared in step (1), 8.8 g of a 20% aqueous sodium hydroxide solution, and 20 mL of *tert*-butanol. The reaction mixture was mixed, heated to 60°C in an oil bath, reacted at 60°C for 2 h, cooled, and added to 150 mL of toluene. The obtained reaction solution was washed, concentrated, and purified to obtain 5.0 g of 2-(4-nitro-2-trifluoromethylphenyl)propionitrile (97% yield).

¹H NMR (CDCl₃, 500 MHz, TMS): δ 8.58 (d, *J* = 2 Hz, 1H), 8.51 (dd, *J₁* = 8.5 Hz, *J₂* = 2 Hz, 1H), 8.00 (d, *J =* 8.5 Hz, 1H), 4.38 (q, *J =* 7 Hz, 1H), 1.72 (d, *J =* 7 Hz, 3H).

¹³C NMR (CDCl₃, 125 MHz): δ 147.3, 142.9, 131.0, 129.1 (q, *J =* 33 Hz), 127.6, 122.6 (q, *J =* 273 Hz), 122.1 (q, *J =* 5 Hz), 119.9, 28.1, 21.9.

### Step (3) Preparation of 1-(4-nitro-2-trifluoromethylphenyl)ethan-1-one

To a 50 mL reaction flask were sequentially added 1.1 g of potassium carbonate, 2.2 g of water, 1 g of 2-(4-nitro-2-(trifluoromethylphenyl)propionitrile prepared in step 2 above, and 10 mL of N, N-dimethylformamide. The atmosphere in the reaction flask was replaced with oxygen, and the reaction mixture was heated to 60°C in an oil bath, reacted at 60°C for 2 h, cooled, and added to 80 mL of toluene. The obtained reaction solution was washed, concentrated, and purified to obtain 0.4 g of 1-(4-nitro-2-trifluoromethylphenyl)ethan-1-one (42% yield).

¹H NMR (CDCl₃, 500 MHz, TMS): δ 8.58 (d, *J =* 1.5 Hz, 1H), 8.48 (dd, *J₁* = 8.5 Hz, *J₂* = 1.5 Hz, 1H), 7.66 (d, *J* = 8.5 Hz, 1H), 2.63 (s, 3H).

¹³C NMR (CDCl₃, 125 MHz): δ 199.8, 148.2, 145.7, 128.5 (q, *J =* 33 Hz), 128.4, 126.9, 122.3 (q, *J =* 273 Hz), 122.3 (q, *J =* 6 Hz), 30.5.

### Example 2 Selection of catalyst for oxidation reaction

### Step (1) Preparation of 2-(4-nitro-2-trifluoromethylphenyl)propionitrile

To a 100 mL reaction flask were sequentially added 5.1 g of ethyl 2-cyanopropionate, 5.5 g of potassium carbonate, 5 g of 2-trifluoromethyl-4-nitro-chlorobenzene, and 20 mL of N, N-dimethylformamide. The reaction mixture was heated to 65°C in an oil bath and reacted at 65°C for 6 h. After the reaction was completed, the reaction solution was cooled and added to 150 mL of toluene. The obtained mixed solution was washed, concentrated and purified to obtain 8.4 g of 2-(4-nitro-2-trifluoromethylphenyl)propionitrile (86% yield).

### Step (2) Preparation of 1-(4-nitro-2-trifluoromethylphenyl)ethan-1-one

To a 50 mL reaction flask were sequentially added 1.1 g of potassium carbonate, 2.2 g of water, 1 g of 2-(4-nitro-2-trifluoromethylphenyl)propionitrile, 10 mL of N, N-dimethylformamide, and 0.05 g of catalyst in Table 1. The atmosphere in the reaction flask was replaced with oxygen, and the reaction mixture was heated to at 65°C in an oil bath and reacted at 65°C for 2 h in an oxygen atmosphere. After the reaction was completed, the reaction solution was cooled and added to 50 mL of toluene. The obtained mixed solution was washed, concentrated and purified to obtain the target product. The catalysts and reaction results were shown in Table 1.

**Table 1 Oxidation catalysts**

| No. | Catalyst | Product weight (g) | Reaction yield (%) |
|---|---|---|---|
| 1 | Ferrous chloride | 0.6 | 63 |
| 2 | Ferric chloride | 0.6 | 63 |
| 3 | Cuprous chloride | 0.9 | 94 |
| 4 | Cupric chloride | 0.8 | 84 |

### Example 3 Preparation of 1-(4-nitro-2-trifluoromethylphenyl)ethan-1-one

### Step (1) Preparation of 2-(4-nitro-2-trifluoromethylphenyl)propionitrile

To a 100 mL reaction flask were sequentially added 51 g of ethyl 2-cyanopropionate, 50 g of 2-trifluoromethyl-4-nitro-chlorobenzene, 55 g of potassium carbonate, 4.9 g of triethylenediamine, and 200 mL of N, N-dimethylformamide. The reaction mixture was heated to 80°C in an oil bath and reacted at 80°C for 13 h. The reaction solution was concentrated, cooled, and added to 100 mL of toluene. The obtained reaction solution was washed, concentrated and purified to obtain product 53 g of 2-(4-nitro-2-trifluoromethylphenyl)propionitrile (98% yield).

### Step (2) Preparation of 1-(4-nitro-2-trifluoromethylphenyl)ethan-1-one

To a 50 mL reaction flask were sequentially added 0.4 g of sodium hydroxide, 10 g of 2-(4-nitro-2-trifluoromethylphenyl)propionitrile, 0.3 g of CuCl, and 25 mL of dimethyl sulfoxide. The atmosphere in the reaction flask was replaced with oxygen, and the reaction mixture was heated to 65°C in an oil bath, reacted at 65°C for 4 h, cooled, and added to 100 mL of toluene. The obtained reaction solution was washed, concentrated and purified to obtain product 8.7 g of 1-(4-nitro-2-trifluoromethylphenyl)ethan-1-one (91% yield).

### Example 4 Preparation of 1-(4-nitro-2-trifluoromethylphenyl)ethan-1-one

### Step (1) Preparation of 2-(4-nitro-2-trifluoromethylphenyl)propionitrile

To a 100 mL reaction flask were sequentially added 4.2 g of ethyl 2-cyanopropionate, 2.7 g of sodium acetate, 5 g of 2-trifluoromethyl-4-nitro-chlorobenzene, and 10 mL of dimethyl sulfoxide. The reaction mixture was heated to 50°C in an oil bath and reacted at 50°C for 5 h. After the reaction was completed, the obtained reaction solution was cooled and added to 100 mL of toluene. The obtained mixed solution was washed, concentrated and purified to obtain 3.5 g of 2-(4-nitro-2-trifluoromethylphenyl)propionitrile (65% yield).

### Step (2) Preparation of 1-(4-nitro-2-trifluoromethylphenyl)ethan-1-one

To a 50 mL reaction flask were sequentially added 3.5 g of 2-(4-nitro-2-trifluoromethylphenyl)propionitrile, 0.1 g of cuprous chloride, and 10 mL of N, N-dimethylformamide. The reaction mixture was heated to at 80°C in an oil bath, added dropwise with 2.0 g of hydrogen peroxide (30 wt.%) under stirring, and reacted under stirring at 80°C for 4 h. After the reaction was completed, the obtained reaction solution was cooled and added to 50 mL of toluene. The obtained mixed solution was washed, concentrated, and purified to obtain 1.6 g of product (48% yield).

### Example 5 Preparation of 1-(4-nitro-2-trifluoromethylphenyl)ethan-1-one

### Step (1) Preparation of 2-(4-nitro-2-(trifluoromethylphenyl)propionitrile

To a 100 mL reaction flask were sequentially added 5.6 g of ethyl 2-cyanopropionate, 4.7 g of sodium carbonate, 5 g of 2-trifluoromethyl-4-nitro-chlorobenzene, and 10 mL of N-methyl pyrrolidone. The reaction mixture was heated to 50°C in an oil bath and reacted at 50°C for 5 h. After the reaction was completed, the reaction solution was cooled and added to 100 mL of toluene. The obtained mixed solution was washed, concentrated, and purified to obtain 5 g of 2-(4-nitro-2-trifluoromethylphenyl)propionitrile (92% yield).

### Step (2) Preparation of 1-(4-nitro-2-trifluoromethylphenyl)ethan-1-one

To a 50 mL reaction flask were sequentially added 0.1 g of sodium hydroxide, 13mL of dimethyl sulfoxide, 5 g of 2-(4-nitro-2-trifluoromethylphenyl)propionitrile, and 0.25 g of cuprous chloride. The reaction mixture was heated to 60°C in an oil bath and reacted at 60 °C by blowing air for 24 h. The reaction solution was cooled and added to 100 mL of toluene. The obtained mixed solution was washed, concentrated, and purified to obtain 3.8 g of product (79% yield).

### Example 6 Preparation of 1-(4-nitro-2-trifluoromethylphenyl)ethan-1-one

### Step (1) Preparation of 2-(4-nitro-2-trifluoromethylphenyl)propionitrile

To a 500 mL reaction flask were sequentially added 51 g of ethyl 2-cyanopropionate, 50 g of 2-trifluoromethyl-4-nitro-chlorobenzene, 55 g of potassium carbonate, 4.9 g of triethylenediamine, and 200 mL of N, N-dimethylformamide. The reaction mixture was heated to 80°C in an oil bath and reacted at 80°C for 13 h. The reaction solution was concentrated, cooled, and added to 100 mL of toluene. The obtained reaction solution was washed, concentrated and purified to obtain product 53 g of 2-(4-nitro-2-trifluoromethylphenyl)propionitrile (98% yield).

### Step (2) Preparation of 1-(4-nitro-2-trifluoromethylphenyl)ethan-1-one

To a 50 mL reaction flask were sequentially added 10 g of 2-(4-nitro-2-trifluoromethylphenyl)propionitrile, 0.2 g of CuCl, and 25 mL of N, N-dimethylformamide. The atmosphere in the reaction flask was replaced with oxygen, and the reaction mixture was heated to 65°C in an oil bath and reacted at 65°C for 14 h. The reaction solution was cooled and added to 100 mL of toluene. The obtained mixed solution was washed, concentrated, and purified to obtain product 7.6 g of 1-(4-nitro-2-trifluoromethylphenyl)ethan-1-one (80% yield).

### Example 7 Preparation of 1-(4-nitro-2-trifluoromethylphenyl)ethan-1-one in "one-pot" manner

To a 100 mL reaction flask were sequentially added 5.1 g of ethyl 2-cyanopropionate, 5.5 g of potassium carbonate, 5 g of 2-trifluoromethyl-4-nitro-chlorobenzene, and 20 mL of N, N-dimethylformamide. The reaction mixture was heated to 60°C in an oil bath and reacted at 60°C for 6 h. After the reaction was completed, 3.1 g of potassium carbonate and 0.1 mg of CuCl were added to the reaction flask. The atmosphere in the reaction flask was replaced with oxygen, and the reaction flask was placed in the oil bath and reacted at 65°C for 2 h. After the reaction was completed, the reaction solution was cooled and added to 150 mL of toluene. The obtained mixed solution was washed, concentrated, and purified to obtain 4.9 g of 1-(4-nitro-2-trifluoromethylphenyl)ethan-1-one (95% yield).

### Example 8 Preparation of 1-(4-nitro-2-trifluoromethylphenyl)ethan-1-one in "one-step" manner

To a 100 mL reaction flask were sequentially added 5.6 g of ethyl 2-cyanopropionate, 9.2 g of potassium carbonate, 5 g of 2-trifluoromethyl-4-nitro-chlorobenzene, 20 mL of N, N-dimethylformamide, and 0.5 mg of CuCl. The atmosphere in the reaction flask was replaced with oxygen, and the reaction mixture was heated to 65°C in an oil bath and reacted at 65°C for 7 h. The reaction solution was cooled and added to 150 mL of toluene. The obtained mixed solution was washed, concentrated, and purified to obtain 3.1 g of 1-(4-nitro-2-trifluoromethylphenyl)ethan-1-one (60% yield).

### Example 9 Preparation of 1-(4-(4-chlorophenoxy)-2-trifluoromethylphenyl) ethan-1-one

To a 25 mL reaction flask were sequentially added 0.5 g of 1-(4-nitro-2-trifluoromethylphenyl)ethan-1-one, 0.3 g of *p*-chlorophenol, 0.2 g of potassium carbonate, and 2.5 mL of N, N-dimethylformamide. The reaction mixture was heated to 125°C in an oil bath and reacted at 125°C for 5 h. After the reaction was completed, the reaction solution was cooled and added to 50 mL of toluene. The obtained mixed solution was washed, concentrated, and purified to obtain 0.57 g of product (85% yield).

### Example 10 Preparation of mefentrifluconazole

0.7 g of water and 3.9 g of dimethyl sulfate were added to a 50 mL three-mouth reaction flask, heated to 33°C, added dropwise with 2.0 g of dimethyl sulfide, and stirred for 15 min to obtain a reaction mixture. 6.3 g of 1-(4-(4-chlorophenoxy)-2-trifluoromethylphenyl)ethan-1-one prepared in Example 9 was added to the reaction mixture at 35°C, then 4.5 g of potassium hydroxide (85 wt.%) was added at 35~45°C under stirring followed by stirring at 38°C for 2 h until raw materials disappeared. 30g of water was added into the obtained mixed solution at 60°C followed by stirring for 20 min. The lower organic product was separated and dissolved in 30 g of dimethyl formamide (DMF). Dimethyl sulfide was removed by distillation to obtain 30 g DMF solution of 2-[4-(4-chlorophenoxy)-2-(trifluoromethyl)phenyl]-2-methyloxirane, which contained 6.6 g product (99% yield) according to quantitative HPLC analysis.

30 g of DMF solution including 6.6 g of 2-[4-(4-chlorophenoxy)-2-(trifluoromethyl)phenyl]-2-methyloxirane was heated to 60°C, then sequentially added with 1.7 g of 1, 2, 4-triazole (99 wt.%) and 0.3 g of sodium hydroxide under stirring, heated to 125°C, and then stirred at 125°C for 5 h. After the reaction was completed, most of the DMF was evaporated under reduced pressure to obtain a concentrated reaction mixture. 30 g of toluene and 20 g of water were added to the concentrated reaction mixture following by separating aqueous phase at 60°C and washing again with 20 g of water. The aqueous phase was separated, and the toluene solution was concentrated under reduced pressure into a solution containing about 50% of the product following by heating, dissolving solids at about 80°C, slowly cooling to 0°C under stirring, and stirring continuously at 0°C for 30 min. Then the filtration was performed to obtain a filter cake, and the filter cake was washed twice with 10 g of toluene precooled to 0°C, and then dried to obtain 6.6 g of 2-[4-(4-chlorophenoxy)-2-(trifluoromethyl)phenyl]-2-methyloxirane (84% yield).

### Efficacy test

Test standard: NY/T 1155.1-2006 Pesticides guidelines for laboratory bioactivity tests. Part 1: Petri dish test for herbicide bioactivity

Targets: *Echinochloa crusgalli (L.) Beauv, Panicum acroanthum Steud., Solanum nigrum L., Chenopodium album L.*

Test agent: ethyl 2-(4-nitro-2-trifluoromethylphenyl)-2-cyanopropanoate prepared in Example 1

The test agent was weighed and dissolved in DMF to obtain a mother liquor. different amounts of mother liquor were weighed and diluted into water to prepare different doses of the test agent.

### Test method: Petri dish test

1 test agent, 1 CK (clean water control), 4 targets, and 2 replicate tests per treatment were required. Test method includes the following steps: putting two filter papers on a Petri dish with a diameter of 9 cm; adding 8 mL of the prepared test agent of different concentrations; putting 10 uniform weed seeds; and then placing in an incubator (30°C/25°C, light for 12 h / for dark 12 h). After treatment, the growth state of the test target was regularly observed, the comprehensive growth inhibition rate of each agent was visually investigated with CK about 7 days after the treatment, and the number of seedlings, stem length and root length of plants were measured, and the mergence rate, stem length, and root length and inhibition rate were calculated compared with CK. The results were shown in Tables 2-5.

**Table 2 Test results of herbicide bioactivity of Solanum nigrum L. (7 days after treatment - inhibition rate %)**

| Agent concentration | *Solanum nigrum L.* | | |
|---|---|---|---|
| | Emergence rate | Stem length | Root length |
| 500 | 100.0 | 100.0 | 100.0 |
| 1000 | 100.0 | 100.0 | 100.0 |

**Table 3 Test results of herbicide bioactivity of Chenopodium album L. (7 days after treatment - inhibition rate %)**

| Agent concentration | *Chenopodium album L.* | | |
|---|---|---|---|
| | Emergence rate | Stem length | Root length |
| 100 | 63.6 | 70.4 | 100.0 |
| 500 | 100.0 | 100.0 | 100.0 |
| 1000 | 100.0 | 100.0 | 100.0 |

**Table 4 Test results of herbicide bioactivity of Panicum acroanthum Steud. (7 days after treatment - inhibition rate %)**

| Agent concentration | *Panicum acroanthum Steud.* | | |
|---|---|---|---|
| | Emergence rate | Stem length | Root length |
| 500 | 100.0 | 100.0 | 100.0 |
| 1000 | 100.0 | 100.0 | 100.0 |

**Table 5 Test results of herbicide bioactivity of Echinochloa crusgalli (L.) Beauv. (7 days after treatment - inhibition rate %)**

| Agent concentration | *Echinochloa crusgalli (L.) Beauv* | | |
|---|---|---|---|
| | Emergence rate | Stem length | Root length |
| 500 | 100.0 | 100.0 | 100.0 |
| 1000 | 100.0 | 100.0 | 100.0 |

## Claims

1. A method for preparing a compound of formula (I), comprising:
(i) reacting a compound (II) with a compound (III) in the presence of a base A to obtain a compound (IV);
(ii) converting the compound (IV) into a compound (V) in the presence of a base B; and
(iii) reacting the compound (V) with an oxidant to obtain the compound of formula (I); as shown in the following reaction scheme: 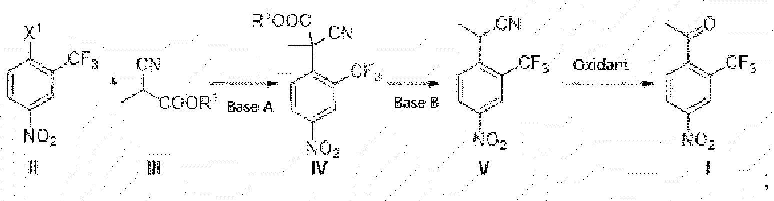
wherein X¹ is halogen; R¹ is hydrogen, a C₁-C₁₀ alkyl group, a C₆-C₁₂ aryl group or a heteroaryl group containing one or two atoms selected from nitrogen, oxygen, and sulfur; and the alkyl group, aryl group and heteroaryl group are unsubstituted or substituted with halogen;
the base A and base B are each independently selected from the group consisting of an alkali metal carbonate, an alkali metal bicarbonate, an alkali metal phosphate, an alkali metal C₁-C₆ alkyl carboxylate, an alkali metal formate, an alkali metal hydroxide, an alkali metal alcoholate, an alkali metal hydride, an alkaline earth metal carbonate, an alkaline earth metal bicarbonate, an alkaline earth metal phosphate, an alkaline earth metal C₁-C₆ alkyl carboxylate, an alkaline earth metal formate, an alkaline earth metal hydroxide, an alkaline earth metal alcoholate, an alkaline earth metal hydride, an alkali metal alkylide, an alkali metal amide compound, an organic amine compound, and a mixture thereof; and
the oxidant is selected from the group consisting of oxygen, air, ozone, peroxide compound, hypohalite, halate, perhalogenate, and a mixture thereof.

2. The method according to claim 1, **characterized in that** the base A and base B are each independently selected from the group consisting of an alkali metal carbonate, an alkali metal phosphate, an alkali metal hydroxide, an organic tertiary amine compound, and a mixture thereof; and the oxidant is selected from the group consisting of oxygen, air, peroxide compound, and a mixture thereof.

3. The method according to claim 2, **characterized in that** the base A and base B are each independently selected from the group consisting of potassium carbonate, potassium hydroxide, sodium carbonate, sodium hydroxide, an alkyl tertiary amine compound, and a mixture thereof; and the oxidant is oxygen or air.

4. The method according to claim 1, **characterized in that** the method is carried out in a "one-pot" manner with the compound (II) and compound (III) as starting materials in the presence of the oxidant, the base A and/or the base B.

5. The method according to claim 1, **characterized in that** the compound (II), the compound (III), the base A and the oxidant are directly mixed to prepare the compound of formula (I), as shown in the following reaction scheme: 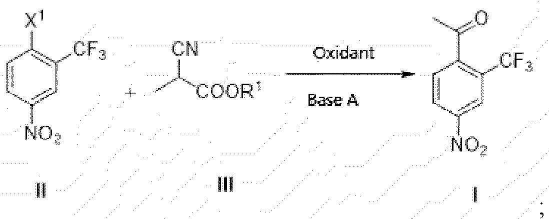 wherein X¹, R¹, the base A and the oxidant are defined as claim 1.

6. The method according to claim 1, **characterized in that** the step (i) is carried out in the presence of a catalyst A; the step (iii) is carried out in the presence of a catalyst B; the catalyst A and the catalyst B are each independently selected from the group consisting of transition elements in groups VIII, IB and IIB and cation thereof, and a mixture thereof.

7. The method according to claim 6, **characterized in that** the catalyst A is an IB-group transition element or cation thereof; and the catalyst B is a transition element in group VIII or IB, or cation thereof.

8. The method according to claim 7, **characterized in that** the catalyst A and the catalyst B are each independently elemental copper, Cu⁺, Cu²⁺, or a mixture thereof.

9. A compound of formula (IV): 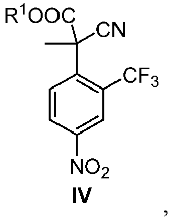 **characterized in that** R¹ is a C₁-C₁₀ alkyl group, a C₆-C₁₂ aryl group or a heteroaryl group containing one or two atoms selected from nitrogen, oxygen and sulfur, wherein the alkyl group, aryl group and heteroaryl group are unsubstituted or substituted with halogen; and R¹ is preferably a C₁-C₄ alkyl group or a C₁-C₄ halogenated alkyl group.

10. Use of the compound (IV) according to claim 9 in the control of *Solanum nigrum L., Chenopodium album L., Echinochloa crusgalli (L.) Beauv, and Panicum acroanthum Steud.*

11. Use of the method according to claim 1, **characterized in that** the compound of formula (I) is reacted with *p*-chlorophenol in the presence of a base D to prepare 1-(4-(4-chlorophenoxy)-2-trifluoromethylphenyl)ethan-1-one, as shown in following reaction scheme:

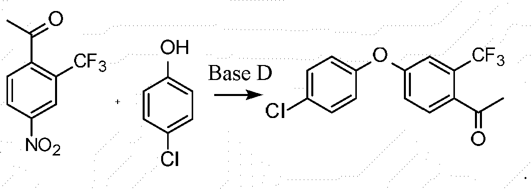

12. The use of claim 11, wherein the base D is selected an alkali metal carbonate or an alkaline earth metal carbonate.

## Patentansprüche

1. Verfahren zur Herstellung einer Verbindung der Formel (I), umfassend:
(i) Umsetzen einer Verbindung (II) mit einer Verbindung (III) in der Gegenwart einer Base A, um eine Verbindung (IV) zu erhalten;
(ii) Umwandeln der Verbindung (IV) in eine Verbindung (V) in der Gegenwart einer Base B; und
(iii) Umsetzen der Verbindung (V) mit einem Oxidationsmittel, um die Verbindung der Formel (I) zu erhalten; wie in dem folgenden Reaktionsschema gezeigt: 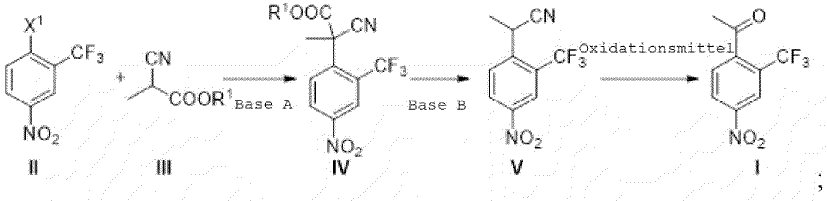
wobei X¹ Halogen ist; R¹ Wasserstoff, eine C₁-C₁₀-Alkylgruppe, eine C₆-C₁₂-Arylgruppe oder eine Heteroarylgruppe enthaltend ein oder zwei Atome ausgewählt aus Stickstoff, Sauerstoff und Schwefel ist; und die Alkylgruppe, Arylgruppe und Heteroarylgruppe unsubstituiert oder mit Halogen substituiert sind;
die Base A und Base B jeweils unabhängig voneinander ausgewählt sind aus der Gruppe bestehend aus einem Alkalimetallcarbonat, einem Alkalimetallbicarbonat, einem Alkalimetallphosphat, einem Alkalimetall-C₁-C₆-Alkylcarboxylat, einem Alkalimetallformiat, einem Alkalimetallhydroxid, einem Alkalimetallalkoholat, einem Alkalimetallhydrid, einem Erdalkalimetallcarbonat, einem Erdalkalimetallbicarbonat, einem Erdalkalimetallphosphat, einem Erdalkalimetall-C₁-C₆-Alkylcarboxylat, einem Erdalkalimetallformiat, einem Erdalkalimetallhydroxid, einem Erdalkalimetallalkoholat, einem Erdalkalimetallhydrid, einem Alkalimetallalkylid, einer Alkalimetallamidverbindung, einer organischen Aminverbindung und einer Mischung davon; und
das Oxidationsmittel ausgewählt ist aus der Gruppe bestehend aus Sauerstoff, Luft, Ozon, Peroxidverbindung, Hypohalogenit, Halogenat, Perhalogenat und einer Mischung davon.

2. Verfahren nach Anspruch 1, **dadurch gekennzeichnet, dass** die Base A und Base B jeweils unabhängig voneinander ausgewählt sind aus der Gruppe bestehend aus einem Alkalimetallcarbonat, einem Alkalimetallphosphat, einem Alkalimetallhydroxid, einer organischen tertiären Aminverbindung und einer Mischung davon; und das Oxidationsmittel ausgewählt ist aus der Gruppe bestehend aus Sauerstoff, Luft, Peroxidverbindung und einer Mischung davon.

3. Verfahren nach Anspruch 2, **dadurch gekennzeichnet, dass** die Base A und Base B jeweils unabhängig voneinander ausgewählt sind aus der Gruppe bestehend aus Kaliumcarbonat, Kaliumhydroxid, Natriumcarbonat, Natriumhydroxid, einer tertiären Alkylaminverbindung und einer Mischung davon; und das Oxidationsmittel Sauerstoff oder Luft ist.

4. Verfahren nach Anspruch 1, **dadurch gekennzeichnet, dass** das Verfahren in einer "Eintopf"-Weise mit der Verbindung (II) und Verbindung (III) als Ausgangsmaterialien in der Gegenwart des Oxidationsmittels, der Base A und/oder der Base B durchgeführt wird.

5. Verfahren nach Anspruch 1, **dadurch gekennzeichnet, dass** die Verbindung (II), die Verbindung (III), die Base A und das Oxidationsmittel direkt gemischt werden, um die Verbindung der Formel (I) herzustellen, wie in dem folgenden Reaktionsschema gezeigt: 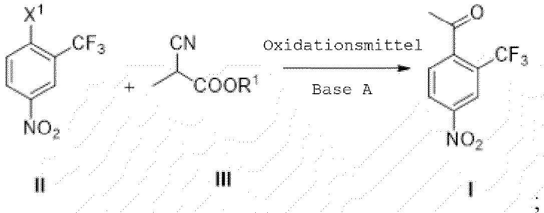 wobei X¹, R¹, die Base A und das Oxidationsmittel wie in Anspruch 1 definiert sind.

6. Verfahren nach Anspruch 1, **dadurch gekennzeichnet, dass** der Schritt (i) in der Gegenwart eines Katalysators A durchgeführt wird; der Schritt (iii) in der Gegenwart eines Katalysators B durchgeführt wird; der Katalysator A und der Katalysator B jeweils unabhängig voneinander ausgewählt sind aus der Gruppe bestehend aus Übergangselementen in Gruppen VIII, IB und IIB und Kation davon, und einer Mischung davon.

7. Verfahren nach Anspruch 6, **dadurch gekennzeichnet, dass** der Katalysator A ein IB-Gruppen-Übergangselement oder Kation davon ist; und der Katalysator B ein Übergangselement in Gruppe VIII oder IB, oder Kation davon ist.

8. Verfahren nach Anspruch 7, **dadurch gekennzeichnet, dass** der Katalysator A und der Katalysator B jeweils unabhängig voneinander elementares Kupfer, Cu⁺, Cu²⁺ oder eine Mischung davon sind.

9. Verbindung der Formel (IV): 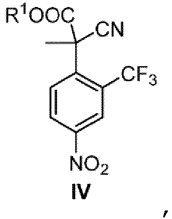 **dadurch gekennzeichnet, dass** R¹ eine C₁-C₁₀-Alkylgruppe, eine C₆-C₁₂-Arylgruppe oder eine Heteroarylgruppe enthaltend ein oder zwei Atome ausgewählt aus Stickstoff, Sauerstoff und Schwefel ist, wobei die Alkylgruppe, Arylgruppe und Heteroarylgruppe unsubstituiert oder mit Halogen substituiert sind; und R¹ vorzugsweise eine C₁-C₄-Alkylgruppe oder eine halogenierte C₁-C₄-Alkylgruppe ist.

10. Verwendung der Verbindung (IV) nach Anspruch 9 bei der Bekämpfung von *Solanum nigrum L., Chenopodium album L., Echinochloa crusgalli (L.) Beauv und Panicum acroanthum Steud.*

11. Verwendung des Verfahrens nach Anspruch 1, **dadurch gekennzeichnet, dass** die Verbindung der Formel (I) mit *p-*Chlorphenol in der Gegenwart einer Base D umgesetzt wird, um 1-(4-(4-Chlorphenoxy)-2-trifluormethylphenyl)ethan-1-on herzustellen, wie in folgendem Reaktionsschema gezeigt:

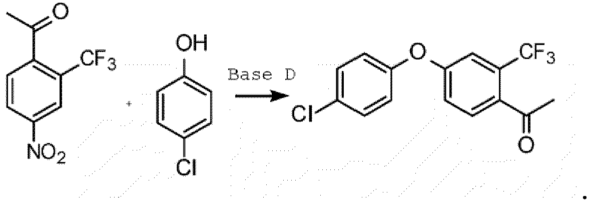

12. Verwendung nach Anspruch 11, wobei die Base D ausgewählt ist aus einem Alkalimetallcarbonat oder einem Erdalkalimetallcarbonat.

## Revendications

1. Procédé de préparation d'un composé de formule (I), comprenant :
(i) la réaction d'un composé (II) avec un composé (III) en présence d'une base A pour obtenir un composé (IV) ;
(ii) la conversion du composé (IV) en un composé (V) en présence d'une base B ; et
(iii) la réaction du composé (V) avec un oxydant pour obtenir le composé de formule (I) ; comme représenté dans le schéma réactionnel suivant : 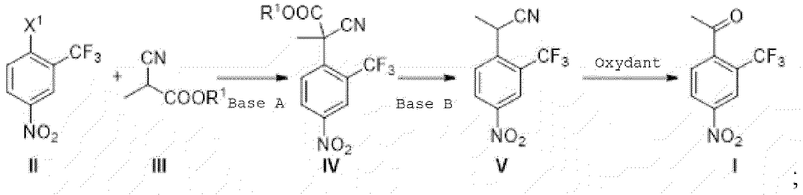
dans lequel X¹ est halogène ; R¹ est hydrogène, un groupe alkyle en C₁-C₁₀, un groupe aryle en C₆-C₁₂ ou un groupe hétéroaryle contenant un ou deux atomes choisis parmi l'azote, l'oxygène et le soufre ; et le groupe alkyle, le groupe aryle et le groupe hétéroaryle sont non substitués ou substitués par halogène ;
la base A et la base B sont chacune choisies indépendamment parmi le groupe constitué d'un carbonate de métal alcalin, d'un bicarbonate de métal alcalin, d'un phosphate de métal alcalin, d'un alkylcarboxylate en C₁-C₆ de métal alcalin, d'un formiate de métal alcalin, d'un hydroxyde de métal alcalin, d'un alcoolate de métal alcalin, d'un hydrure de métal alcalin, d'un carbonate de métal alcalino-terreux, d'un bicarbonate de métal alcalino-terreux, d'un phosphate de métal alcalino-terreux, d'un alkylcarboxylate en C₁-C₆ de métal alcalino-terreux, d'un formiate de métal alcalino-terreux, d'un hydroxyde de métal alcalino-terreux, d'un alcoolate de métal alcalino-terreux, d'un hydrure de métal alcalino-terreux, d'un alkylure de métal alcalin, d'un composé amide de métal alcalin, d'un composé amine organique et d'un mélange de ceux-ci ; et
l'oxydant est choisi parmi le groupe constitué de l'oxygène, de l'air, de l'ozone, d'un composé peroxyde, de l'hypohalite, de l'halate, du perhalogénate et d'un mélange de ceux-ci.

2. Procédé selon la revendication 1, **caractérisé en ce que** la base A et la base B sont chacune choisies indépendamment parmi le groupe constitué d'un carbonate de métal alcalin, d'un phosphate de métal alcalin, d'un hydroxyde de métal alcalin, d'un composé amine tertiaire organique et d'un mélange de ceux-ci ; et l'oxydant est choisi parmi le groupe constitué de l'oxygène, de l'air, d'un composé peroxyde et d'un mélange de ceux-ci.

3. Procédé selon la revendication 2, **caractérisé en ce que** la base A et la base B sont chacune choisies indépendamment parmi le groupe constitué du carbonate de potassium, de l'hydroxyde de potassium, du carbonate de sodium, de l'hydroxyde de sodium, d'un composé amine tertiaire alkylée et d'un mélange de ceux-ci ; et l'oxydant est l'oxygène ou l'air.

4. Procédé selon la revendication 1, **caractérisé en ce que** le procédé est réalisé de manière « monopot » avec le composé (II) et le composé (III) comme matières premières en présence de l'oxydant, de la base A et/ou de la base B.

5. Procédé selon la revendication 1, **caractérisé en ce que** le composé (II), le composé (III), la base A et l'oxydant sont directement mélangés pour préparer le composé de formule (I), comme représenté dans le schéma réactionnel suivant : 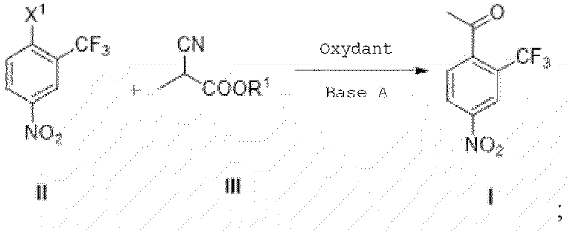 dans lequel X¹, R¹, la base A et l'oxydant sont définis selon la revendication 1.

6. Procédé selon la revendication 1, **caractérisé en ce que** l'étape (i) est réalisée en présence d'un catalyseur A ; l'étape (iii) est réalisée en présence d'un catalyseur B ; le catalyseur A et le catalyseur B sont chacun indépendamment choisis parmi le groupe constitué d'éléments de transition des groupes VIII, IB et IIB et de leurs cations, et d'un mélange de ceux-ci.

7. Procédé selon la revendication 6, **caractérisé en ce que** le catalyseur A est un élément de transition du groupe IB ou un cation de celui-ci ; et le catalyseur B est un élément de transition du groupe VIII ou IB ou un cation de celui-ci.

8. Procédé selon la revendication 7, **caractérisé en ce que** le catalyseur A et le catalyseur B sont chacun indépendamment du cuivre élémentaire, Cu⁺, Cu²⁺, ou un mélange de ceux-ci.

9. Composé de formule (IV) : 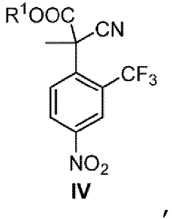 **caractérisé en ce que** R¹ est un groupe alkyle en C₁-C₁₀, un groupe aryle en C₆-C₁₂ ou un groupe hétéroaryle contenant un ou deux atomes choisis parmi l'azote, l'oxygène et le soufre, dans lequel le groupe alkyle, le groupe aryle et le groupe hétéroaryle sont non substitués ou substitués par halogène ; et R¹ est de préférence un groupe alkyle en C₁-C₄ ou un groupe alkyle halogéné en C₁-C₄.

10. Utilisation du composé (IV) selon la revendication 9 dans le contrôle de *Solanum nigrum L., Chenopodium album L., Echinochloa crusgalli (L.) Beauv et Panicum acroanthum Steud.*

11. Utilisation du procédé selon la revendication 1, **caractérisée en ce que** le composé de formule (I) réagit avec le *p*-chlorophénol en présence d'une base D pour préparer la 1-(4-(4-chlorophénoxy)-2-trifluorométhylphényl)éthan-1-one, comme représenté dans le schéma réactionnel suivant :

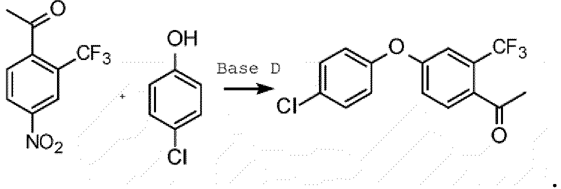

12. Utilisation selon la revendication 11, dans laquelle la base D est choisie comme carbonate de métal alcalin ou carbonate de métal alcalino-terreux.
